# EUROPÄISCHE PATENTSCHRIFT

(11) **EP 0 276 723 B1**
(45) Veröffentlichungstag und Bekanntmachung des Hinweises auf die Patenterteilung: **08.12.1993**
(21) Anmeldenummer: 88100647.2
(22) Anmeldetag: 19.01.1988
(51) Int. Cl.: C07K 13/00, C12Q 1/68, G01N 33/53

(54) **Vorläuferprotein des APC-Polypeptids, dafür codierende DNA und diagnostische Verwendung der DNA und des Proteins**
Precursor protein of APC polypeptide, DNA coding therefor and diagnostic use of the DNA and protein
Précurseur protéinique de polypeptide-APC, ADN le codant et utilisation diagnostique de cet ADN et de cette protéine

(30) Priorität: 30.01.1987 DE 3702789
(43) Veröffentlichungstag der Anmeldung: 03.08.1988
(73) Patentinhaber: BAYER AG, 51368 Leverkusen (DE)
(72) Erfinder: Müller-Hill, Benno, Prof. Dr., D-5000 Köln (DE); Kang, Jie, Dipl.-Biol., D-5300 Bonn 2 (DE); Lemaire, Hans-Georg, Dipl.-Biol., D-5000 Köln 1 (DE); Unterbeck, Axel, Dr., West Haven, CT 06516 (US)

(56) Entgegenhaltungen:
- EP-A- 0 274 826
- US-A- 4 666 829
- NATURE, Band 325, 19. Februar 1987; J. KANG et al., Seiten 733-736#
- SCIENCE, Band 235, 20. Februar 1987; D. GOLDGABER et al., Seiten 880-887#
- PROCEEDINGS OF THE NATL. ACADEMY OF SCIENCES USA, Band 84, Juni 1987; N.K. ROBAKIS et al.; Seiten 4190-4194#
- PROCEEDINGS OF THE NATL. ACADEMY OF SCIENCES USA, Band 83, April 1986; A. ROHER et al., Seiten 2662-2666#
- PROCEEDINGS OF THE NATL. ACADEMY OF SCIENCES USA, Band 82, Juni 1985; C.L. MASTERS et al.; Seiten 4245-4249#
- CHEMICAL ABSTRACTS, Band 104, 1986, Columbus, OH (US); C.L. MASTERS et al., Seite 506, Nr. 127641f#

## Beschreibung

Die vorliegende Erfindung betrifft das Vorläuferprotein des "amyloid plaque core" (APC)-Polypeptids, Fragmente des Vorläuferproteins sowie die diagnostische Verwendung des Vorläuferproteins bzw. der Fragmente. Weiterhin betrifft die Erfindung die für das Vorläuferprotein codierende DNA, Fragmente dieser DNA sowie die diagnostische Verwendung der DNA bzw. der Fragmente.

Die Alzheimersche Krankheit wurde im Jahre 1907 von dem deutschen Neurologen Alois Alzheimer zum ersten Mal als eine eigenständige klinische und pathologische Erscheinung beschrieben (Alzheimer, A. (1907) Zentralblatt für Nervenheilkunde und Psychiatrie, 177-179). Sie ist die häufigste degenerative Gehirnerkrankung bei alten Menschen. Allein in Amerika leiden ungefähr 2 Millionen Menschen heute an der Krankheit und mindestens 100 000 davon sterben jedes Jahr (Wurtman, R.J. (1985) Sci.Am. 252, 48-56).

Die Krankheit tritt beim Menschen zwischen dem 40. und 80. Lebensjahr auf. Die Betroffenen verlieren allmählich ihr Gedächtnis und ihre Konzentrationsfähigkeit. Der Zustand des geistigen Verfalls steigert sich innerhalb von 3 bis 10 Jahren, bis die Patienten weder sprechen, denken noch für sich sorgen können und schließlich sterben. Die Ursache für diese Demenz ist unbekannt. Es gibt weder eine definitive Diagnose noch eine Therapie.

Bei Hirnautopsien von an der Alzheimerschen Krankheit gestorbenen Menschen sind im Mikroskop typische Veränderungen zu erkennen.
- Die Zahl der Neuronen hat abgenommen, vor allem in den Scheitellappen, also in den Teilen des Gehirns, wo die Gedächtnisleistungen lokalisiert sind. Ein Verlust an Neuronen, die normalerweise Acetylcholin freisetzen, ist ebenfalls deutlich sichtbar.
- Weiterhin kommen im cerebralen Cortex drei äußerst ungewöhnliche Strukturen vor, die im Gehirn gesunder Menschen nicht existieren und daher zur Diagnose (nach dem Tod) benutzt werden:
   1) intrazelluläre Neurofibrillen (NFTs, "neurofibrillary tangles")
      Im Zellkörper von Neuronen der Großhirnrinde und des Hippocampus findet man Bündel, die aus zwei helixartig umeinandergedrehten Filamenten (PHFs, "paired helical filaments") bestehen.
   2) extraxelluläre amyloide Plaques (APC, "amyloid plaque core")
      Die neuritischen Plaques enthalten Amyloid und die Überreste abgestorbener Zellen, sie sind in der Großhirnrinde, dem Hippocampus und dem Mandelkern verstreut. Die Anzahl der Plaques korreliert mit dem Grad des geistigen Verfalls.
   3) cerebrovasculäres Amyloid (ACA, "amyloid congophilic angiopathy")
      Als Amyloid bezeichnet man eine proteinreiche Masse. Solche formlosen Proteinaggregate sind ringsum die Blutgefäße bzw. in der Wand von Blutgefäßen im Gehirn zu finden.

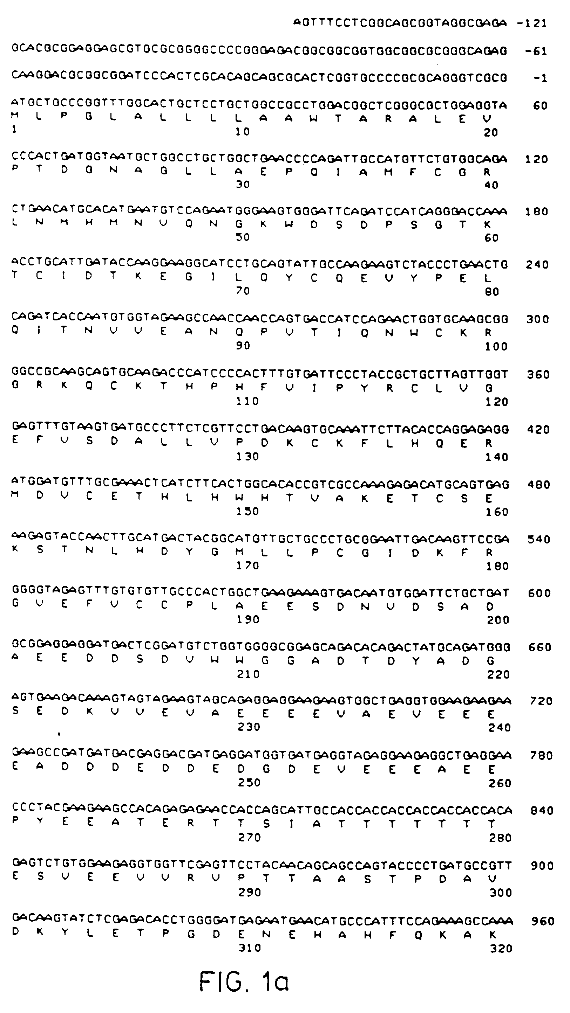

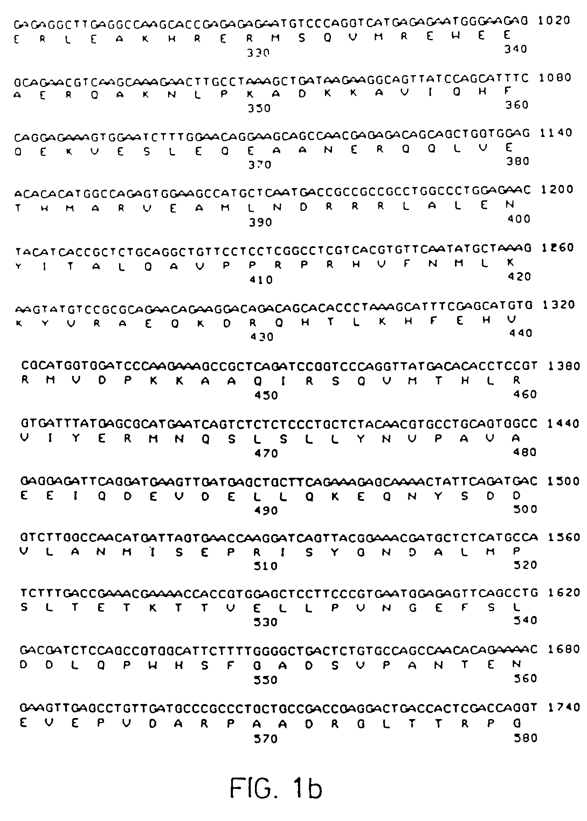

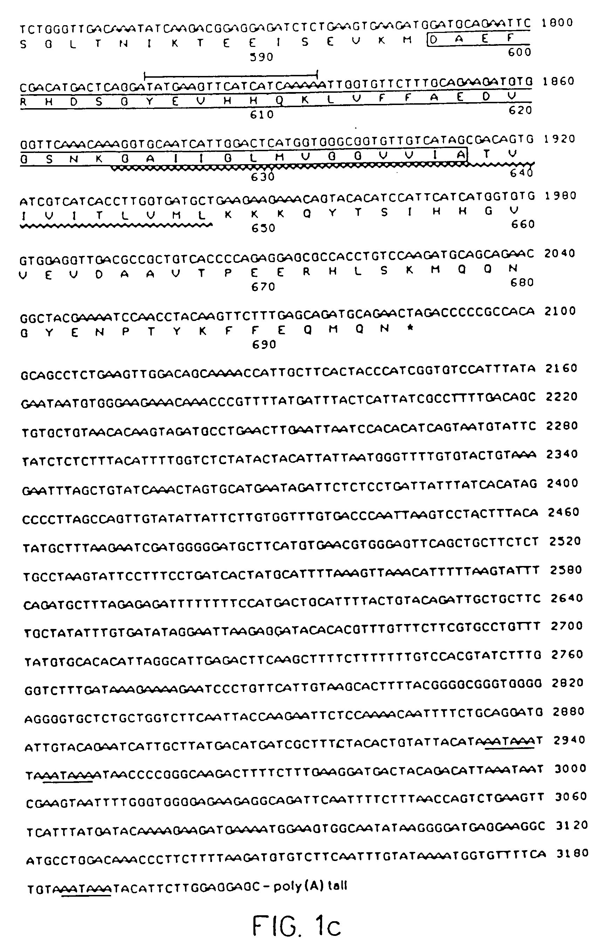

Die Proteinkomponente des ACA wurde isoliert und sequenziert (Glenner, G.G. & Wong, C.W. (1984) Biochem. Biophys. Res. Commun 120, 885-890). Die Aminosäuresequenz hat keine Homologie zu bekannten Proteinsequenzen. Die Proteinkomponenten der PHFs bzw. APC wurden ebenfalls isoliert und sequenziert (Masters, C.L., Multhaupt, G., Simms, G., Pottgiesser, J., Martins R.N. and Beyreuther, K. (1985) EMBO 4, 2757-2763 und Masters, C.L., Simms, G., Weinman, N.A., Multhaupt, G., McDonald, B.L. and Beyreuther, K. (1985) Proc. Natl. Acad. Sci. USA 82, 4245-4249). Nach den Aminosäuresequenzen handelt es sich wahrscheinlich in allen drei Fällen um das gleiche Polypeptid mit einem Molekulargewicht von 4,5 kD. Die entsprechende Sequenz ist in der Fig. 1 a-c eingerahmt (Position 597-638).

Es gibt mehrere Hypothesesn um die Herkunft dieses APC-Proteins zu erklären. Es könnte ein normales Protein im Gehirn (oder auch in einem anderen Organ) sein, bei dem entweder die Regulation der Biosynthese entgleist oder der physiologische Abbau gestört ist. Die Anhäufungen in sehr großer Menge könnten dann die Ursache der Krankheit sein. Falls es ein abnormes Protein ist und seine ungewöhnliche Aggregationsfähigkeit die Krankheit verursacht, könnte es ebenfalls von einem gesunden, menschlichem Gen kodiert werden, das durch irgendeinen Faktor, z.B. Viren, Nahrungsmittel oder Umweltgifte falsch gesteuert würde. Der Fehler könnte auch in einer Modifikation der ursprünglichen Protein-Vorstufe bestehen. Andererseits könnte aber auch ein virales Gen für die Synthese des APC-Proteins verantwortlich sein.

Es wurde nun versucht die Herkunft und Natur des APC-Proteins zu klären, dessen Aggregation im cerebralen Cortex eines der biochemischen Hauptsymptome von Alzheimer-Patienten ist, um damit ein Werkzeug zur verbesserten Diagnostik der Alzheimer-Krankheit zu erhalten.

Dazu wurde eine fötale, humane Hirn c-DNA-Bank mit pA+mRNA des cerebralen Cortex konstruiert.

Die c-DNA-Synthese wurde nach Okayama und Berg (Okayama, H. and Berg, P. Mol. Cell. Biol. 2, 161-170 (1982); Okayama, H. and Berg, P. Mol. Cell. Biol. 3, 280-289 (1983)) durchgeführt und die c-DNA in E. coli HB 101 transformiert (Aviv, H. and Leder,P. Proc. Natl. Acad. Sci. USA 69, 1408 (1972)). Jede der so erhaltenen c-DNA-Banken enthält mehr als 1 x 10⁶ unabhängige c-DNA-Klone.

Für das Sichten der Bank wurde eine DNA-Sonde benutzt, deren Sequenz aus der Sequenz des APC-Polypeptids abgeleitet war. Es wurde die Sequenz gewählt, die den Aminosäuren in Position 10 - 16 des APC entspricht. Die entsprechende Sequenz ist in der Fig. 1 c durch eine Klammer markiert (Position 1815-1835). Um eine optimale Hybridisierung zu gewährleisten, wurde die Degeneration des genetischen Codes berücksichtigt und als Sonde ein Gemisch mit der folgenden Sequenz 
    T  A  A     T CA
5'TT TG TG TGCAC T  TA3'   ,
    C  G  G     C GG
hergestellt und benutzt. Dies ist ein 64fach degeneriertes 20-mer. Die entsprechende Sequenz ist in der Fig. 1 c durch eine Klammer markiert (Position 1815-1835). Ein Test von 100 000 c-DNA Klonen der menschlichen fötalen cerebralen Cortex Bank führte zu der Isolierung eines vollständigen (full-length) c-DNA-Klons mit der Serien-Nr. EC 9.110, der ein Protein kodiert, welches die APC-Sequenz enthält und somit das Vorläufer-Protein des APC-Peptids darstellt. Die Sequenz der c-DNA und die Aminosäuresequenz des kodierten Proteins sind der Figur (1) zu entnehmen. Die Sequenzanalyse erfolgte nach der Dideoxy-Methode (Sanger, F., Nicklen, S. and Coulson, A.R. Proc. Natl. Acad. Sci. USA 74, 5463-5467 (1977) und Guidelines for quick and simple Plasmid Sequencing, Handbook, (1986) Boehringer Mannheim GmbH, Biochemica, D-6800 Mannheim). Über die natürliche Funktion des APC-Vorläuferproteins ist zur Zeit noch nichts bekannt.

Die vorliegende Erfindung betrifft also die Desoxyribonucleinsäure der Sequenz gemäß Fig. 1 und deren funktionellen Äquivalente. Der Ausdruck funktionelle Äquivalente bedeutet in diesem Zusammenhang, daß innerhalb der Sequenz, bedingt durch die Degeneration des genetischen Code, einzelne Nucleotide ausgetauscht oder auch derivatisiert werden können, ohne daß dies einen Einfluß auf die Funktion der Nukleinsäure hat. Insbesondere betrifft die Erfindung die DNA der Sequenz gemäß Fig. 1 von Position 1 bis Position 2089 und deren funktionellen Äquivalente. Dieser Teil der DNA ist der Teil, der für das Vorläuferprotein codiert. Durch einige Besonderheiten in der Sequenz ist das Protein und die korrespondierende DNA-Sequenz ein interessantes Werkzeug für die Diagnose der Alzheimer Krankheit auf molekularer Ebene. Hier ist insbesondere der Bereich von etwa Position 600 bis etwa Position 900 erwähnenswert. Dieser Teil codiert für eine ungewöhnlich große Zahl von sauren Aminosäuren, bezogen auf die Länge dieses Abschnitts. Ganz besonders auffällig sind auch die sieben Threonine (Position: DNA 819 -840 / Aminosäuren 274 - 280) hintereinander. Für die Entwicklung von DNA-Sonden für die Diagnostik sind solche Bereiche besonders interessant, da sie durch ihre außergewöhnliche Sequenz einzigartig sind und somit eine hochspezifische Detektion erlauben. Die Erfindung betrifft auch Fragmente der DNA aus Fig. 1 bzw. aus dieser DNA abgeleitete Oligonukleotide sowie deren Verwendung als Sonden in der Diagnostik. Für Hybridisierungsexperimente wird die DNA nicht in der vollen Länge eingesetzt. Normalerweise benutzt man für Hybridisierungen Fragmente einer Länge von etwa 10 bis 50 Nukleotiden. Längere Fragmente führen meist zu Problemen bei der Handhabung. Fragmente mit weniger als 10 Nukleotiden besitzen meistens nicht die hinreichende Spezifität bzw. die Bindung ist zu schwach.

Die DNA gemäß Fig. 1 bzw. die Fragmente dieser DNA können sehr gut bei der Diagnose der Alzheimer-Erkankung verwendet werden, zum Feststellen von Mutationen wie z.B. von Deletionen, Insertionen und Punktmutationen oder Rearrangement-Fehlern.

Die vorliegende Erfindung ermöglicht die Diagnostik der Alzheimer-Erkrankung auf molekularer Ebene. Dies gilt gleichermaßen für die Präsymptomatische Diagnostik der Alzheimer-Erkrankungen. Durchgeführt werden kann die Analytik mit bekannten Techniken aus der DNA-Technologie wie z.B. den von Antonarkais et al (1985), in Hum. Gen 69, 1-14 beschriebenen Techniken.

Zu der vorliegenden Erfindung gehört auch das von der DNA codierte Vorläuferprotein bzw. dessen Fragmente. Der Nachweis dieses Proteins bzw. der Fragmente stellt ebenfalls einen Ansatzpunkt für die Diagnose der Alzheimer Krankheit dar. Auch hier sind die Besonderheiten der Sequenz (Aminosäuren: ca. Position 200 bis ca. Position 290) von besonderer Bedeutung. Fragmente des Vorläuferproteins, insbesondere aus der Region 200 bis 290, können sehr gut als antigene Peptide für die Herstellung von polyklonalen oder monoklonalen Antikörpern verwendet werden, die wiederum Verwendung in der Diagnostik finden.

Funktionelle Äquivalente im Zusammenhang mit dem Protein bzw. den Peptiden bedeutet, daß sowohl in der Sequenz des Proteins als auch bei den Peptiden Variationen in Form von Aminosäureaustauschen bzw. Derivatisierungen möglich sind, die keinen Einfluß auf die Funktion dieser Peptide z.B. als Antigen haben.

### Legende zu Figur 1 a-c.

Nukleotid-Sequenz 5ʹ → 3ʹ des c-DNA-Klons, der für das Vorläuferprotein des APC-Polypeptids codiert und die aus der DNA abgeleitete Aminosäuresequenz. Die Aminosäuren sind mit dem folgenden Einbuchstaben-Code bezeichnet:

### Aminosäuren

- A: Ala Alanin
- B: Asz Asn oder Asp
- C: Cys Cystein (Cystin)
- D: Asp Asparaginsäure
- E: Glu Glutaminsäure
- F: Phe Phenylalanin
- G: Gly Glycerin
- H: His Histidin
HS Homoserin
HSL Homoserinlacton
- I: Ile Isoleucin
- K: Lys Lysin
- L: Leu Leucin
- M: Met Methionin
- N: Asn Asparagin
Nle Norleucin
- P: Pro Prolin
- Q: Gln Glutamin
- R: Arg Arginin
- S: Ser Serin
- T: Thr Threonin
- V: Val Valin
- W: Trp Tryptophan
- Y: Tyr Tyrosin
- Z: Glx Glu oder Gln
- X: nicht identifiziert

## Patentansprüche (Patentansprüche für folgende(n) Vertragsstaat(en): AT, BE, CH, DE, FR, GB, IT, LI, NL, SE)

1. Desoxyribonukleinsäure der Sequenz gemäß Fig. 1a - 1c und solche funktionellen Equivalente dieser Sequenz. in denen. bedingt durch die Degeneration des genetischen Codes, einzelne Nukleotide ausgetauscht oder derivatisiert sind, ohne daß dies einen Einfluß auf die Funktion der Nukleinsäure hat.

2. Desoxyribonukleinsäure der Sequenz von Position 1 bis Position 2089 gemäß Fig. 1a - 1c und solche funktionellen Equivalente dieser Sequenz, in denen. bedingt durch die Degeneration des genetischen Codes, einzelne Nukleotide ausgetauscht oder derivatisiert sind, ohne daß dies einen Einfluß auf die Funktion der Nukleinsäure hat.

3. Desoxyribonukleinsäure der Sequenz von Position 600 bis 900 aus Fig. 1a - 1c und solche funktionellen Equivalente dieser Sequenz, in denen, bedingt durch die Degeneration des genetischen Codes, einzelne Nukleotide ausgetauscht oder derivatisiert sind, ohne daß dies einen Einfluß auf die Funktion der Nukleinsäure hat.

4. Verwendung der Desoxyribonukleinsäure gemäß den Ansprüchen 1 bis 3 zur in vitro Diagnose der Alzheimer-Erkrankung.

5. Testkit enthaltend die Desoxyribonukleinsäure gemäß den Ansprüchen 1 bis 3.

6. Vorläuferprotein des Amyloiden Plaque Proteins der Sequenz gemäß Fig. 1a - 1c und solche funktionellen Equivalente dieser Sequenz, in denen, bedingt durch die Degeneration des genetischen Codes, einzelne Aminosäuren ausgetauscht oder derivatisiert sind, ohne daß dies einen Einfluß auf die Funktion des Proteins hat.

7. Protein der Sequenz von Position 210 bis Position 290 gemäß Fig. 1a - 1c und solche funktionellen Equivalente, in deren Sequenz, bedingt durch die Degeneration des genetischen Codes, einzelne Aminosäuren ausgetauscht oder derivatisiert sind, ohne daß dies einen Einfluß auf die Funktion des Proteins hat.

8. Fragmente der Proteine nach den Ansprüchen 6 und 7.

9. Verwendung der Proteine bzw. Fragmente gemäß den Ansprüchen 6 bis 8 zur in vitro Diagnose der Alzheimer Erkrankung.

10. Nicht therapeutische Verwendung der Proteine bzw. Fragmente gemäß den Ansprüchen 6 bis 8 als Antigene zur Herstellung von Antikörpern.

11. Antikörper gerichtet gegen die Proteine bzw. Fragmente gemäß den Ansprüchen 6 bis 8.

12. Testkit enthaltend die Antikörper gemäß Anspruch 11.

13. Verwendung der Antikörper gemäß Anspruch 11 zur in vitro Diagnose der Alzheimer Erkrankung.

## Patentansprüche (Patentansprüche für folgende(n) Vertragsstaat(en): ES, GR)

1. Verwendung der Desoxyribonukleinsäure gemäß Fig. 1a - 1c und solche funktionellen Equivalente dieser Sequenz, in denen, bedingt durch die Degeneration des genetischen Codes, einzelne Nukleotide ausgetauscht oder derivatisiert sind, ohne daß dies einen Einfluß auf die Funktion der Nukleinsäure hat, zur in vitro Diagnose der Alzheimer-Erkrankung.

2. Verwendung der Desoxyribonukleinsäure der Sequenz von Position 1 bis Position 2089 gemäß Fig. 1a - 1c und solche funktionellen Equivalente dieser Sequenz, in denen, bedingt durch die Degeneration des genetischen Codes, einzelne Nukleotide ausgetauscht oder derivatisiert sind, ohne daß dies einen Einfluß auf die Funktion der Nukleinsäure hat, zur in vitro Diagnose der Alzheimer-Erkrankung.

3. Verwendung der Desoxyribonukleinsäure der Sequenz von Position 600 bis Position 900 aus Fig. 1a - 1c und solche funktionellen Equivalente dieser Sequenz, in denen, bedingt durch die Degeneration des genetischen Codes, einzelne Nukleotide ausgetauscht oder derivatisiert sind, ohne daß dies einen Einfluß auf die Funktion der Nukleinsäure hat, zur in vitro Diagnose der Alzheimer-Erkrankung.

4. Verwendung des Vorläuferproteins des Amyloiden Plaque Proteins der Sequenz gemäß Fig. 1a - 1c und solche funktionellen Equivalente dieser Sequenz, in denen, bedingt durch die Degeneration des genetischen Codes, einzelne Aminosäuren ausgetauscht oder derivatisiert sind, ohne daß dies einen Einfluß auf die Funktion des Proteins hat, zur in vitro Diagnose der Alzheimer-Erkrankung.

5. Verwendung der Proteine bzw. Fragmente gemäß den Ansprüchen 1 bis 4 als Antigene zur Herstellung von Antikörpern.

6. Verwendung der Antikörper gemäß Anspruch 5 zur in vitro Diagnose der Alzheimer-Erkrankung.

## Claims (Claims for the following Contracting State(s): AT, BE, CH, DE, FR, GB, IT, LI, NL, SE)

1. Deoxyribonucleic acid of the sequence shown in Figures 1a - 1c and those functional equivalents of the sequence in which, due to the degeneration of the genetic code, individual nucleotides are exchanged or derivatised without this having an effect on the function of the nucleic acid.

2. Deoxyribonucleic acid of the sequence from position 1 to position 2089 as shown in Figures 1a - 1c and those functional equivalents of this sequence in which, due to the degeneration of the genetic code, individual nucleotides are exchanged or derivatised without this having an effect on the function of the nucleic acid.

3. Deoxyribonucleic acid of the sequence from position 600 to position 900 as shown in Figures 1a - 1c and those functional equivalents of this sequence in which, due to the degeneration of the genetic code, individual nucleotides are exchanged or derivatised without this having an effect on the function of the nucleic acid.

4. Use of the deoxyribonucleic acid according to Claims 1 to 3 for the in vitro diagnosis of Alzheimer's disease.

5. Test kit containing the deoxyribonucleic acid according to Claims 1 to 3.

6. Precursor protein of amyloid plaque protein of the sequence shown in Figures 1a - 1c and those functional equivalents of this sequence in which, due to the degeneration of the genetic code, individual amino acids are exchanged or derivatised without this having an effect on the function of the protein.

7. Protein of the sequence from position 210 to position 290 as shown in Figures 1a - 1c and those functional equivalents in whose sequence, due to the degeneration of the genetic code, individual amino acids are exchanged or derivatised without this having an effect on the function of the protein.

8. Fragments of the proteins according to Claims 6 and 7.

9. Use of the proteins or fragments according to Claims 6 to 8 for the in vitro diagnosis of Alzheimer's disease.

10. Non-therapeutic use of the proteins or fragments according to Claims 6 to 8 as antigens for the production of antibodies.

11. Antibodies directed against the proteins or fragments according to Claims 6 to 8.

12. Test kit containing the antibodies according to Claim 11.

13. Use of the antibodies according to Claim 11 for the in vitro diagnosis of Alzheimer's disease.

## Claims (Claims for the following Contracting State(s): ES, GR)

1. Use of the deoxyribonucleic acid according to Figures 1a - 1c and those functional equivalents of this sequence in which, due to the degeneration of the genetic code, individual nucleotides are exchanged or derivatised without this having an effect on the function of the nucleic acid, for the in vitro diagnosis of Alzheimer's disease.

2. Use of the deoxyribonucleic acid of the sequence from position 1 to position 2089 as shown in Figures 1a -1c and those functional equivalents of this sequence in which, due to the degeneration of the genetic code, individual nucleotides are exchanged or derivatised without this having an effect on the function of the nucleic acid, for the in vitro diagnosis of Alzheimer's disease.

3. Use of the deoxyribonucleic acid of the sequence from position 600 to position 900 as shown in Figures 1a - 1c and those functional equivalents of this sequence in which, due to the degeneration of the genetic code, individual nucleotides are exchanged or derivatised without this having an effect on the function of the nucleic acid, for the in vitro diagnosis of Alzheimer's disease.

4. Use of the precursor protein of amyloid plaque protein of the sequence shown in Figures 1a - 1c and those functional equivalents of this sequence in which, due to the degeneration of the genetic code, individual amino acids are exchanged or derivatised without this having an effect on the function of the protein, for the in vitro diagnosis of Alzheimer's disease.

5. Use of the proteins or fragments according to Claims 1 to 4 as antigens for the production of antibodies.

6. Use of the antibodies according to Claim 5 for the in vitro diagnosis of Alzheimer's disease.

## Revendications (Revendications pour l'(les) Etat(s) contractant(s) suivant(s): AT, BE, CH, DE, FR, GB, IT, LI, NL, SE)

1. Acide désoxyribonucléique de séquence suivant les figures 1a-1c et équivalents fonctionnels de cette séquence dans lesquels, par dégénérescence du code génétique, certains nucléotides sont échangés ou dérivatisés sans que cela ait une influence sur la fonction de l'acide nucléique.

2. Acide désoxyribonucléique de séquence allant de la position 1 à la position 2089 conformément aux figures 1a-1c et équivalents fonctionnels de cette séquence dans lesquels, du fait de la dégénérescence du code génétique, certains nucléotides sont échangés ou dérivatisés sans que cela exerce une influence sur la fonction de l'acide nucleique.

3. Acide désoxyribonucléique de séquence allant de la position 600 à la position 900 sur les figures 1a-1c et les équivalents fonctionnels de cette séquence dans lesquels, du fait de la dégénérescence du code génétique, certains nucléotides sont échangés ou dérivatisés sans que cela exerce une influence sur la fonction de l'acide nucléique.

4. Utilisation de l'acide désoxyribonucléique suivant les revendications 1 à 3 pour le diagnostic in vitro de la maladie d'Alzheimer.

5. Kit analytique contenant l'acide désoxyribonucléique suivant les revendications 1 à 3.

6. Précurseur protéique de la protéine de la plaque amyloïde de séquence suivant les figures 1a-1c et les équivalents fonctionnels de cette séquence dans lesquels, du fait de la dégénérescence du code génétique, des amino-acides individuels sont échangés ou sont dérivatisés sans que cela exerce une influence sur la fonction de la protéine.

7. Protéine de la séquence allant de la position 210 à la position 290 conformément aux figures 1a-1c et les équivalents fonctionnels de cette séquence dans lesquels, du fait de la dégénérescence du code génétique, des amino-acides individuels sont échangés ou dérivatisés sans que cela exerce une influence sur la fonction de la protéine.

8. Fragments des protéines suivant les revendications 6 et 7.

9. Utilisation des protéines ou des fragments suivant les revendications 6 à 8 pour le diagnostic in vitro de la maladie d'Alzheimer.

10. Utilisation des protéines ou des fragments suivant les revendications 6 à 8 comme antigènes non thérapeutiques pour la préparation d'anticorps.

11. Anticorps dirigés contre les protéines ou fragments suivant les revendications 6 à 8.

12. Kit analytique contenant les anticorps suivant la revendication 11.

13. Utilisation des anticorps suivant la revendication 11 pour le diagnostic in vitro de la maladie d'Alzheimer.

## Revendications (Revendications pour l'(les) Etat(s) contractant(s) suivant(s): ES, GR)

1. Utilisation de l'acide désoxyribonucléique suivant les figures 1a-1c et équivalents fonctionnels de cette séquence dans lesquels, sous l'effet de la dégénérescence du code génétique, certains nucléotides sont échangés ou dérivatisés sans que cela ait une influence sur la fonction de l'acide nucléique, pour le diagnostic in vitro de la maladie d'Alzheimer.

2. Utilisation de l'acide désoxyribonucléique de la séquence allant de la position 1 à la position 2089 suivant les figures 1a-1c et des équivalents fonctionnels de cette séquence dans lesquels, du fait de la dégénérescence du code génétique, certains nucléotides sont échangés ou dérivatisés sans que cela exerce une influence sur la fonction de l'acide nucléique, pour le diagnostic in vitro de la maladie d'Alzheimer.

3. Utilisation de l'acide désoxyribonucléique de la séquence allant de la position 600 à la position 900 sur les figures 1a-1c et des équivalents fonctionnels de cette séquence dans lesquels, à cause de la dégénérescence du code génétique, certains nucléotides sont échangés ou dérivatisés sans que cela ait une influence sur la fonction de l'acide nucléique, pour le diagnostic in vitro de la maladie d'Alzheimer.

4. Utilisation du précurseur protéique de la protéine de la plaque amyloïde de la séquence suivant les revendications 1a-1c et des équivalents fonctionnels de cette séquence dans lesquels, à cause de la dégénérescence du code génétique, certains amino-acides sont échangés ou dérivatisés sans que cela exerce une influence sur la fonction de la protéine, pour le diagnostic in vitro de la maladie d'Alzheimer.

5. Utilisation des protéines ou des fragments suivant les revendications 1 à 4 comme antigènes pour la production d'anticorps.

6. Utilisation des anticorps suivant la revendication 5 pour le diagnostic in vitro de la maladie d'Alzheimer.
